Europäisches Patentamt

European Patent Office

Office européen des brevets

(11) Veröffentlichungsnummer: **0 441 274 A2**

# (12) EUROPÄISCHE PATENTANMELDUNG

(21) Anmeldenummer: 91101409.0

(22) Anmeldetag: 02.02.91

(51) Int. Cl.5: **C07K 3/22**

(30) Priorität: 08.02.90 DE 4003773

(43) Veröffentlichungstag der Anmeldung:
**14.08.91 Patentblatt 91/33**

(84) Benannte Vertragsstaaten:
**AT BE CH DE DK ES FR GB GR IT LI LU NL SE**

(71) Anmelder: **BEHRINGWERKE Aktiengesellschaft**
**Postfach 1140**
**W-3550 Marburg 1(DE)**

(72) Erfinder: **Römisch, Jürgen, Dr.**
**Am Kähnelplatz 6**
**W-3550 Marburg(DE)**
Erfinder: **Amann, Egon, Dr. c/o Hoechst Japan**
**Ltd Ph.Res.Labs**
**1-3-2 Minamidai**
**Kawagoe, Saitama(JP)**
Erfinder: **Grote, Mathias, Dr.**
**Gladenbacher Weg 65**
**W-3550 Marburg(DE)**

(74) Vertreter: **Klein, Otto, Dr. et al**
**Hoechst AG Zentrale Patentabteilung**
**Postfach 80 03 20**
**W-6230 Frankfurt am Main 80(DE)**

(54) Verfahren zur Reinigung von Lipocortinen.

(57) Es wird ein Verfahren zur Reinigung von Lipocortinen, besonders der gentechnisch hergestellten Proteine rPP4, rPP4-X und rPP4-delta, mittels Behandlung einer Lösung eines Lipocortins mit einem Anionenaustauscher in Gegenwart eines Chelatbildners und eines Detergenzes beschrieben.

EP 0 441 274 A2

## VERFAHREN ZUR REINIGUNG VON LIPOCORTINEN

Die Erfindung betrifft ein Verfahren zur Reinigung von Lipocortinen, besonders der gentechnisch hergestellten Proteine rPP4, rPP4-X und rPP4-delta. Diese Proteine wirken antikoagulatorisch und entzündungshemmend.

Das natürliche Protein PP4 ist in EP-A 0 123 307 beschrieben.

Die gentechnische Herstellung von PP4, einem ebenfalls antikoagulatorisch wirksamen Protein PP4-X sowie einer Deletionsvariante mit erhöhter Aktivität (PP4-delta) ist in DE-A 39 10 240 beschrieben.

Diese Proteine können als einer Gruppe von Proteinen angehörend betrachtet werden, die als Lipocortine (Glucocorticoid-induzierte Phospholipase-hemmende Proteine) oder Calpactine (Calziumabhängige phospholipid-und aktin-bindende Proteine) bezeichnet werden.

Aufgabe der Erfindung ist, ein Verfahren zur Gewinnung und Reinigung von Lipocortinen, besonders der gentechnisch hergestellten Proteine PP4, PP4-X und PP4-delta (rPP4, rPP4-X und rPP4-delta) zur Verfügung zu stellen.

Verfahren zur Reinigung von PP4 sind zwar bekannt, diese sind jedoch zur Gewinnung von gentechnisch hergestellten Lipocortinen wegen der anderen Zusammensetzung der Ausgangslösung unbefriedigend.

Gegenstand der Erfindung ist ein Verfahren zur Reinigung eines Lipocortins durch die Behandlung einer Lösung eines solchen mit einem Anionenaustauscher, dadurch gekennzeichnet, daß dieser Lösung ein Chelatbildner und ein Detergenz zugegeben werden und diese Lösung mit dem Austauscher in Kontakt gebracht und die Flüssigkeit abgetrennt wird.

Der Austauscher kann dann mit einer detergenzfreien Pufferlösung gewaschen und das Lipocortin eluiert werden.

Gegebenenfalls wird jedoch das Lipocortin aus der Flüssigkeit gewonnen.

Das beschriebene Verfahren ist besonders für gentechnisch hergestellte Lipocortine geeignet. Ein solches Lipocortin ist besonders rPP4, rPP4-X oder rPP4-delta. Falls bei Zugabe eines Chelatbildners zu einer Lipocortinlösung ein Niederschlag entsteht, wird dieser vorzugsweise vor Zugabe eines Detergenzes abgetrennt.

Eine ein Lipocortin enthaltende Lösung ist besonders ein E.coli-Lysat, wie sie bei gentechnischen Verfahren unter Verwendung von E.coli anfallen. Das chelatbildende Reagenz wird in einer Konzentration von 0,001-0,05 mol/l, bevorzugt 0,002-0,02 mol/l verwendet. Der pH-Wert wird auf 6,5-9,5, bevorzugt pH 6,8-7,5 eingestellt. Als Detergenzien können ionisch oder nicht-ionische oder zwitterionische verwendet werden, bevorzugt Derivate der Cholsäure und ihre Salze und ᴿTriton X-100 in einer Konzentration von 0,1-2,0 % (allgemein).

Als Ionenaustauscher können basische Ionenaustauscher, bevorzugt Q-, QAE- oder DEAE-ᴿSepharose, -ᴿCellulose, -ᴿSephadex oder -ᴿFractogel (ein Aminogruppen tragendes Copolymerisat aus Pentaerythrit, Methacrylsäure-Derivaten und Polyethylenglykol vernetzt mit Divinylbenzol) oder Hydroxylapatit verwendet werden. Es kann aus einer Pufferlösung von 0,01-0,1 mol/l Tris, HEPES oder Glycin, bei einem pH-Wert von 6,5-9,5 adsorbiert werden.

Während rPP4 und rPP4-delta unter den beschriebenen Bedingungen an den Anionenaustauscher gebunden werden, wird rPP4-X bei pH 6,5-8,0 nicht gebunden. Es wird jedoch eine Anreicherung dieses Proteins erzielt, da ein Großteil verunreinigender Proteine an den Austauscher adsorbiert wird.

Überraschenderweise wurde außerdem gefunden, daß neben rPP4 auch rPP4-X und rPP4-delta an immobilisierte Polysschwefelsäureester von Sacchariden oder trägergebundene sulfatierte Zukker binden, jedoch nur in Anwesenheit von Calzium, bei einer Leitfähigkeit von 0-10 mS und einem pH-Wert von 6,5-9,5; durch Erhöhung der Ionenstärke können die Proteine eluiert werden. Diese Proteine binden nicht an diese Adsorbentien in Abwesenheit von Calzium oder in Gegenwart von chelatbildenden Reagenzien.

Zur Weiterreinigung von rPP4, rPP4-X oder rPP4-delta können diese in Gegenwart von Calzium auch an Kaolin oder ᴿAerosil adsorbiert werden. Elution erfolgt durch Erhöhung der Ionenstärke und/oder mit Hilfe chelatbildender Reagenzien.

Zur weiteren Reinigung dieser Proteine kann auch die Bindung an Calzium-Phosphat verwendet werden. Durch Erhöhung der Ionenstärke oder mittels chelatbildender Reagenzien, wie EDTA oder Salze der Citronensäure oder durch ein diese Reagenzien enthaltenden Gemisch können diese Proteine eluiert werden.

Zur weiteren Reinigung können die Lipocortine einer Affinitätschromatographie unterworfen werden. Dazu können die Lipocortine enthaltenden Lösungen dialysiert, in einem Puffer enthaltend 0,01-0,05 mol/l, bevorzugt 0,02 mol/l Tris-HCl, pH 8,5, nach Zusatz von Calziumionen im Batch-Verfahren an trägergebundenes Heparin adsorbiert, das Adsorbens gewaschen und die Proteine mittels eines Stufengradienten eluiert und anschließend dialysiert werden. Die Dialysate werden mit 0,01-20 mmol/l, bevorzugt 1 mmol/l EDTA versetzt, vorzugsweise nochmals mit trägergebundenem Heparin in Kontakt gebracht und die Lipocortine dialysiert.

Allgemein kann diese Affinitätschromatographie an trägergebundenen Polyschwefelsäureestern von Sacchariden oder sulfatierten Zuckern, beispielsweise trägergebundenem Heparin, Dextransulfat, Heparansulfat, Chondroitinsulfat, Keratansulfat oder Dermatansulfat, bevorzugt Heparin-[R]Sepharose oder -[R]Fractogel oder Dextransulfat-[R]Sepharose oder -[R]Fractogel, aus einer Pufferlösung, die 0,01-0,1 mol/l Tris, HEPES oder Glycin enthält, bei einem pH-Wert von 6,5-9,5, bevorzugt pH 6,8-8,5, die bevorzugt 5 mmol/l Calzium enthält, und durch Elution adsorbierter Proteine durch Erhöhung der Ionenstärke wie bei der Ionenaustauscher-Chromatographie durchgeführt werden. In Abwesenheit von Calzium oder Anwesenheit chelatbildender Reagenzien binden rPP4, rPP4-X und rPP4-delta nicht an diese Adsorbentien.

Zur weiteren Reinigung kann eine Adsorption an [R]Aerosil oder Kaolin durchgeführt werden, besonders an [R]Aerosil 200 (Fa. Serva) oder Kaolin (Fa. Serva) und aus E.coli-Fermentationsmedien (s.o.) oder aus einer Pufferlösung mit einem pH-Wert von 6,5-9,5, die 0,01-0,1 mol/l Tris, HEPES oder Glycin und 1-20 mmol/l Calzium enthält. Adsorbierte Proteine können durch Erhöhung der Ionenstärke wie bei der Ionenaustauscher-Chromatographie und/oder mittels chelatbildender Reagenzien, bevorzugt 1-20 mmol/l EDTA oder 0,01-0,2 mol/l eines Salzes der Citronensäure oder eines Gemisches eluiert werden.

In einer bevorzugten Verfahrensweise werden lipocortinhaltige Lösungen aus E.coli-Fermentationsgemischen oder Pufferlösungen aus 0,01-0,1 mol/l Tris/HCl, oder Glycin und einem pH-Wert von 6,5-9,5 mit Calzium bis zu einer Endkonzentration von 1-10 mmol/l versetzt, mit Kaolin, [R]Aerosil 200 oder Calzium-Phosphat unter Schütteln in Kontakt gebracht, die Flüssigkeiten abgetrennt, das Adsorbens gewaschen und adsorbierte Proteine mittels eines chelatbildenden Reagenzes, bevorzugt EDTA oder Salzen der Citronensäure oder eines Gemisches, eluiert. Nach Dialyse der Eluate kann die weitere Reinigung der Proteine durch Adsorption an ein Heparin-Harz, wie oben beschrieben, fortgesetzt werden.

Adsorption und Elution der Proteine an Calzium-Phosphat erfolgt wie unter Adsorption an [R]Aerosil und Kaolin beschrieben, jedoch ist zur Bindung die Anwesenheit von Calzium nicht erforderlich.

Eine besonders bevorzugte Verfahrensweise ist die Kombination (EDTA/[R]Triton) DEAE Austauscher, danach Heparin-Chromatografie mit Ca$^{2+}$ und danach Heparin-Chromatografie mit EDTA wie in Beispiel 1 beschrieben.

Alle Verfahrensschritte können bei Raumtemperatur ausgeführt werden. Ausnahmen sind gekennzeichnet.

Für die Beschreibung werden folgende Abkürzungen verwendet:
[R]Aerosil 200: Silizium-dioxid
DEAE: Diethylaminoethyl
EDTA: Ethylendiamintetraessigsäure
HEPES: N-2-Hydroxyethylpiperazin-N'-2-Ethansulfonsäure
Kaolin: Hydratisiertes Aluminium-Silicat
PAGE: Polyacrylamid-Gelelektrophorese
PP4: Plazentares Protein 4
Q: Quarternär-Amino
[R]Rivanol: 6,9-Diamino-2-Ethoxy-Acridin-Lactat
SDS: Natriumdodecylsulfat
Tris: Tris(hydroxymethyl)aminomethan
VAC: Vaskuläres Antikoagulans

Die Erfindung wird an nachstehenden Beispielen erläutert:

**Beispiel 1**

a) Solubilisierung phospholipid-assoziierter Proteine

1,0 l zentrifugierter E.coli-Lysate, die 1,0 mg/ml rPP4 oder rPP4-delta enthielten, in 0,05 mol/l Tris/HCl, pH 7,5, 5 mmol/l EDTA, wurden mit [R]Triton X-100 bis zu einer Endkonzentration von 1,0% versetzt, unter Schütteln für 10 min bei Raumtemperatur inkubiert und anschließend zentrifugiert.

b) Ionenaustauscher-Chromatographie

Der Überstand nach Zentrifugation wurde mit 300 ml DEAE-[R]Sepharose ("Harzmaterial") äquilibriert mit 0,02 mol/l Tris/HCl, pH 7,5 (Säulenpuffer 1), unter Rühren für eine Stunde in Kontakt gebracht, die Flüssigkeit durch Filtration abgetrennt, das Adsorbens mit Säulenpuffer 1 gewaschen und das Harzmaterial in eine Säule gefüllt. Elution adsorbierter Proteine erfolgte mit Säulenpuffer 1, der 0,25 mol/l NaCl enthielt.

c) Adsorption an trägergebundenes Heparin (+ Calzium)

Nach Dialyse wurde das Ionenaustauscher-Eluat (300 ml), wenn nötig, auf eine Protein-Konzentration von 0,2-3,0 mg/ml an rPP4 oder rPP4-delta nach Zugabe von CaCl$_2$ auf eine Endkonzentration von 5 mmol/l gebracht, mit 70 ml Heparin-[R]Fractogel äquilibriert mit 0,02 mol/l Tris/HCl, pH 8,5, 5 mmol/l CaCl$_2$ (Säulenpuffer 2), unter Rühren für eine Stunde in Kontakt gebracht, die überstehende Flüssigkeit abfiltriert, das Harz mit Säulenpuffer 2 gewaschen und adsorbierte Proteine durch Erhöhung der Ionenstärke mittels einer 0,3 molaren NaCl-Pufferlösung eluiert.

d) Heparin-Chromatographie (+EDTA)

Nach Dialyse wurde das Heparin-Eluat (100 ml) mit EDTA bis zu einer Endkonzentration von 1 mmol/l versetzt, mit 50 ml Heparin-Harz, äquilibriert mit 0,02 M Tris/HCl, pH 8,5, 1 mM EDTA (Säulenpuffer 3) in einer Säule in Kontakt gebracht und der rPP4- oder rPP4-delta-haltige Durchlauf gegen eine Lösung aus 0,02 M Tris/HCl, pH 6,8-8,5, dialysiert und anschließend lyophilisiert. Nach Lösen der Lyophilisate in entsprechenden Volumina aqua dest. oder Pufferlösungen wurden 95-100% der biologischen Aktivitäten, bezogen auf die Materialien vor Gefriertrocknung, wiedergefunden.

Die Reinheit der rPP4-Heparin-Durchläufe (d) betrug >95%. Die Ausbeute betrug etwa 55%, bezogen auf die Ausgangsmaterialien und etwa 40 % für rPP4-delta.

**Beispiel 2**

a) Solubilisierung phospholipid-assoziierter Proteine

1,0 1 zentrifugierter E.coli-Lysate in 0,02 M Tris/HCl, pH 6,8, die 0,8 mg/l rPP4-X, bei einer Gesamt-Proteinkonzentration von etwa 20 mg/ml enthielt, wurde mit Triton X-100 bis zu einer Endkonzentration von 1,0% versetzt, für 10 min unter Schütteln inkubiert und anschließend zentrifugiert.

b) Ionenaustauscher-Chromatographie

Der überstand nach Zentrifugation wurde mit 300 ml DEAE-Sepharose, äquilibriert mit 0,02 M Tris/HCl, pH 6,8, unter Rühren in Kontakt gebracht, die Flüssigkeit durch Filtration abgetrennt, das Adsorbens gewaschen und das Harzmaterial in eine Säule gefüllt. Elution adsorbierter Proteine erfolgte mit einer Pufferlösung, die 1,5 M NaCl enthielt.

Die nach der Inkubation mit dem Ionenaustauscher-Harz abfiltrierte, rPP4-x-enthaltende Lösung wurde auf einen pH-Wert von 8,5 gebracht, mit 5 mmol/l CaCl$_2$ versetzt, mit trägergebundenem Heparin inkubiert, das Adsorbens mit einer Lösung aus 0,02 M Tris/HCl, pH 8,5, gewaschen, adsorbierte Proteine eluiert, das Eluat dialysiert und nach Zugabe auf 1 mmol/l EDTA nochmals mit Heparin-Harz inkubiert, wie im Beispiel 1, c. und d., beschrieben.

Überraschend war der Befund, daß rPP4-X unter diesen Bedingungen nicht an den Anionenaustauscher band. Trotzdem wurde dadurch ein erheblicher Reinigungseffekt erzielt, da ein Teil der E.coli-Proteine an das Harz adsorbiert wurden. Die Ausbeute lag nach den Heparin-Chromatographien bei etwa 60%, bezogen auf das Ausgangsmaterial, d.h. etwa 480 mg rPP4-X, mit einer Reinheit von >95%.

**Beispiel 3**

Adsorption an Kaolin, $^R$Aerosil 200 oder Calcium-Phosphat

Zentrifugierte E.coli-Lysat-Lösungen aus 0,05 M Tris/ HCl, pH 7,5, die 1,0 mg/ml rPP4, rPP4-X oder rPP4-delta enthielten, bei einem Gesamtproteingehalt von etwa 20 mg/ml, wurden mit 5 mM CaCl$_2$ versetzt und in getrennten Ansätzen jeweils mit 25 mg Kaolin oder $^R$Aerosil 200 pro ml Proteinlösung unter Rühren für eine Stunde inkubiert und das Adsorbens mit einer Lösung aus 0,02 mol/l Tris/ HCl, pH 8,0, gewaschen. Adsorption an Calcium-Phosphat fand unter den gleichen Bedingungen statt, jedoch war die Anwesenheit von Calzium zur Bindung nicht erforderlich. Elution adsorbierter Proteine erfolgte mit einer Lösung aus 0,02 M Tris/HCl, pH 8,0, die 0,02 mol/l EDTA enthielt oder einem Gemisch aus 0,01 M EDTA und 0,1M Natrium-Citrat.

Nach Dialyse der Eluate gegen eine Pufferlösung aus 0,02 M Tris/HCl, pH 8,5, wurde die Reinigung der Proteine durch Adsorption an trägergebundenes Heparin, wie in Beispiel 1, c. und d., beschrieben, fortgesetzt. Die Ausbeuten der >95% reinen Proteine rPP4 und rPP4-X betrugen nach den Heparin-Chromatographien zwischen 50 und 60%, bezogen auf die Ausgangsmaterialien.

Die, wie in den Beispielen beschrieben, gereinigten Proteine rPP4 und rPP4-X wurden auf ihre physikochemischen und biologischen Eigenschaften geprüft. Sie zeigten in allen Eigenschaften Identität mit den entsprechenden aus Plazenta isolierten Proteinen.

**Patentansprüche**

1. Verfahren zur Reinigung eines Lipocortins durch die Behandlung einer Lösung eines solchen mit einem Anionenaustauscher, dadurch gekennzeichnet, daß dieser Lösung ein Chelatbildner und ein Detergenz zugegeben werden und diese Lösung mit dem Austauscher in Kontakt gebracht und die Flüssigkeit abgetrennt wird.

2. Verfahren nach Anspruch 1, dadurch gekennzeichnet, daß der Austauscher mit einer detergenzfreien Pufferlösung gewaschen und das Lipocortin eluiert wird.

3. Verfahren nach Anspruch 1, dadurch gekennzeichnet, daß das Lipocortin aus der Flüssigkeit gewonnen wird.

4. Verfahren nach Anspruch 1, dadurch gekennzeichnet, daß das Lipocortin ein gentechnisch hergestelltes Lipocortin ist.

5. Verfahren nach Anspruch 1, dadurch gekennzeichnet, daß das Lipocortin rPP4, rPP4-X oder rPP4-delta ist.

6. Verfahren nach Anspruch 1, dadurch gekennzeichnet, daß die ein Lipocortin enthaltende Lösung aus einer E.coli-, Hefe-, oder Animalzellen-Kultur stammt.

7. Verfahren nach Anspruch 1, dadurch gekennzeichnet, daß das Lipocortin durch Chromatographie an einem immobilisierten Polyschwefelsäureester eines Saccharids oder an einem trägergebundenen sulfatierten Zucker weiter gereinigt wird.

8. Verfahren nach Anspruch 1, dadurch gekennzeichnet, daß das Lipocortin durch Chromatographie an einem immobilisierten Polyschwefelsäureester eines Saccharids oder an einem trägergebundenen sulfatierten Zucker in Gegenwart von Calciumionen und anschließend in Gegenwart von EDTA weiter gereinigt wird.

9. Verfahren nach Anspruch 1, dadurch gekennzeichnet, daß das Lipocortin durch Adsorption an Kaolin, [R]Aerosil oder Calzium-Phosphat weiter gereinigt wird.